(19) Europäisches Patentamt

European Patent Office

Office européen des brevets

(11) **EP 1 523 346 B1**

(12) **EUROPÄISCHE PATENTSCHRIFT**

(45) Veröffentlichungstag und Bekanntmachung des Hinweises auf die Patenterteilung:
**01.03.2006 Patentblatt 2006/09**

(21) Anmeldenummer: **03765062.9**

(22) Anmeldetag: **19.07.2003**

(51) Int Cl.:
**A61L 33/04** (2006.01)

(86) Internationale Anmeldenummer:
**PCT/EP2003/007913**

(87) Internationale Veröffentlichungsnummer:
**WO 2004/009148 (29.01.2004 Gazette 2004/05)**

(54) **BESCHICHTUNGSZUSAMMENSETZUNG FÜR EINE IMPLANTIERBARE MEDIZINISCHE VORRICHTUNG UND VERFAHREN ZUR BESCHICHTUNG EINER SOLCHEN VORRICHTUNG**

COATING COMPOSITION FOR AN IMPLANTABLE MEDICAL DEVICE AND METHOD FOR COATING SUCH A DEVICE

COMPOSITION DE REVETEMENT POUR UN DISPOSITIF MEDICAL IMPLANTABLE ET PROCEDE POUR LE REVETEMENT D'UN TEL DISPOSITIF

(84) Benannte Vertragsstaaten:
**AT BE BG CH CY CZ DE DK EE ES FI FR GB GR HU IE IT LI LU MC NL PT RO SE SI SK TR**

(30) Priorität: **23.07.2002 DE 10234398**

(43) Veröffentlichungstag der Anmeldung:
**20.04.2005 Patentblatt 2005/16**

(73) Patentinhaber: **LS medcap GmbH**
**72379 Hechingen (DE)**

(72) Erfinder: **SEVASTIANOV, Viktor**
**Moscow, 123182 (RU)**

(74) Vertreter: **Otten, Hajo et al**
**Witte, Weller & Partner**
**Patentanwälte,**
**Postfach 105462**
**70047 Stuttgart (DE)**

(56) Entgegenhaltungen:
**EP-A- 0 496 349**

- LIU X D ET AL: "Chitosan coated cotton fiber: preparation and physical properties" CARBOHYDRATE POLYMERS, APPLIED SCIENCE PUBLISHERS, LTD. BARKING, GB, Bd. 44, Nr. 3, März 2001 (2001-03), Seiten 233-238, XP004219911 ISSN: 0144-8617
- YUKIHIRO OZAKI, KAGEYOSHI ONO, IKUYO SAKAGUCHI, YOSHIKO KATO: "Histological Study of the Accelerating Effect of Shikonin and Alkannin on the Proliferation of Granulation Tissue in Rats" NATURAL MEDICINES, Bd. 56, Nr. 2, April 2002 (2002-04), Seiten 29-33, XP009022471

## EP 1 523 346 B1

**Beschreibung**

Die vorliegende Erfindung betrifft eine Beschichtungszusammensetzung für eine implantierbare medizinische Vorrichtung, wobei die Beschichtung zumindest ein Polymer und zumindest einen biologisch aktiven Wirkstoff aufweist.

Im Stand der Technik sind verschiedene Beschichtungszusammensetzungen und Verfahren zur Beschichtung von implantierbaren medizinischen Vorrichtungen hinreichend bekannt.

Beschichtete implantierbare medizinische Vorrichtungen kommen bspw. als Haut-, Knochen- oder Knorpelersatz zum Einsatz und spielen insbesondere in der Gefäßchirurgie als Prothesen eine große Rolle.

Derartige Prothesen werden in ein Körperlumen, bspw. in ein Blutgefäß, eines Patienten implantiert, um diese Gefäße für die betreffenden Fluide auf eine bestimmte Strecke zu ersetzen - beispielsweise bei einer Gefäßprothese - oder um sie aufzuweiten und offen zu halten durch einen sogenannten Stent. Die meist zylindrisch geformten Prothesen stützen dabei die Gefäßauskleidung und verhindern, dass die Gefäße kollabieren oder dass ihre Auskleidung den Durchlauf durch die Gefäße versperrt. Herkömmlich für Prothesen verwendete Materialien sind bspw. synthetische Materialien wie gewebte Fäden aus Polyethylenterephthalat (PET) oder aus expandiertem Polytetrafluoroethylen (ePTFE), daneben werden aber auch verschiedene Metalle eingesetzt.

Nicht in jedem Fall muss bei einer Gefäßverengung oder einem Gefäßverschluss (Stenose) ein chirurgischer Eingriff von außen her durchgeführt werden. Sogar bei Verschlüssen von Herzkranzgefäßen kann in vielen Fällen eine Herzoperation bei geöffnetem Brustkorb durch eine Prothese vermieden werden, die durch Gefäße (intravasal) eingebracht wird. Hierfür wird beispielsweise über eine Vene ein Katheter bis in die Gefäßverengung eingebracht, welcher an seiner Spitze einen aufblasbaren Ballon und eine aufweitbare mechanische Stütze für das Gefäß (Stent) trägt. Durch das Aufblasen des Ballons werden Gefäß und Stent gleichzeitig aufgeweitet; der Durchfluss von Blut durch die Stenose wird so wieder möglich.

Bei dieser etwas gewaltsamen Aufweitung wird das Gefäß verletzt. Es wird durch den steifen Stent gegen Kollabierung gestützt. Auch wenn der Stent im entfalteten Zustand eine sehr offene Struktur besitzt, gibt es viele Kontaktstellen zwischen dem steifen Stützmaterial und der verletzten Gefäßwand. Diese bringt zwei zeitlich aufeinanderfolgende Probleme:

Alle GefäßVerletzungen wirken thrombogen. Über die Aktivierung der Blutplättchen durch Gefäßmaterial unter dem Endothel bildet sich ein Thrombus im Lumen, der den Austritt von Blut verhindern soll. Auch Fremdoberflächen wie Metalle oder Kunststoffe, aus welchen Stents hergestellt werden, wirken thrombogen und können durch die Thrombusbildung innerhalb kurzer Zeit zu einem erneuten Gefäßverschluss (Restenose) führen.

Bei der Heilung des durch die Aufweitung geschädigten Gefäßes kommt es ferner zu einer Narbenbildung insbesondere dort, wo durch Druckstellen des steifen Stents ein permanenter Stress auf das sich regenerierende Gewebe ausgeübt wird. Überschießende Proliferation von Narbengewebe führt bei ca. 30 % aller unbeschichteten Stents nach längerer Zeit zu einer Restenose.

Die Beschichtung der als Stützmaterial notwendigerweise steifen Stents soll beide Risiken der Restenose verhindern - die kurzfristige Thrombengefahr wie die langfristige Proliferation von Narbengewebe.

Diese Probleme, nämlich das Vermeiden der Koagulation und Proliferation und das Verhindern der Aktivierung der Blutplättchen werden im Stand der Technik verschiedenartig angegangen. Ziel solcher Forschungen sind bspw. Beschichtungen von Stents, die eine erhöhte Hemokompatibilität bieten sollen. Seit längerer Zeit werden bspw. in die Beschichtung der Stents antikoagulierende, antimikrobielle, antiinflammatorische oder antiproliferative Agenzien eingesetzt, welche im allgemeinen als "biologisch aktive" Substanzen bezeichnet werden. Diese Substanzen sollen aus dem Beschichtungsmaterial des Stents derart freigesetzt werden, dass sie Entzündungen des umliegenden Gewebes, überschießendes. Wachstum der glatten Muskelzellen oder das Verklumpen von Blut verhindern.

Aus der US 5,788,979 ist ein Verfahren zur Beschichtung eines biologisch kompatiblen Materials bekannt, welches mit dem Blut eines Patienten in Kontakt kommt, wobei durch die Zusammensetzung der Beschichtung eine Koagulation des Blutes durch das Biomaterial verhindert werden soll. In dem Verfahren wird zunächst ein biologisch abbaubares Material, welches mit dem Blut und Gewebe des menschlichen Körpers kompatibel ist, in einem flüssigen Status vorbereitet und anschließend ein antikoagulierendes Mittel in das flüssige, biologisch abbaubare Material gegeben. Dadurch wird ein flüssiges Beschichtungsmaterial hergestellt, welches auf ein biologisch kompatibles Material auf kontinuierliche Art und Weise aufgetragen und anschließend getrocknet werden kann. Mit diesem Verfahren und den verwendeten Materialien können Schichtdicken von weniger als 100 $\mu$m produziert werden.

In der US 5,788,979 wird weiterhin offenbart, dass das biologisch abbaubare Material insbesondere ein biologisch abbaubares, synthetisches Polymer sein kann, wie bspw. Polyglykolsäuren, Polymilchsäuren, Polyhydroxybutyrate, Polyhydroxyvalerate, Polydioxanone, modifizierte Stärken, Zellulosen usw.

Darüber hinaus wird vorgeschlagen, dass neben dem anti-koagulierenden Mittel weitere Substanzen in der Beschichtung vorliegen können, wie bspw. anti-inflammatorische, antiproliferative, antibiotische Substanzen. Als Beispiele hierzu werden in der Patentschrift Dexamethason, Gentamycin und Hirudin aufgeführt.

Nachteilig an den genannten Beschichtungen ist, dass bei Verwendung von anti-koagulierenden Mitteln die jeweilige Verabreichung und Dosierung sorgfältigst beobachtet und auf den einzelnen Patienten abgestimmt werden muss, da bei diesen Substanzen immer eine große Gefahr von akuten Blutungen besteht. Daneben entwickeln zahlreiche Patienten nach Kontakt mit rekombinant produziertem Hirudin Antikörper gegen Hirudin-Thrombin-Komplexe, wodurch der anti-thrombotische Effekt dieser Substanz unkontrollierbar wird. Die Verwendung von Glucocorticoiden wie Dexamethason ruft insbesondere über längere Zeiträume die bei diesen Hormonen üblicherweise beobachteten Nebenwirkungen auf den Protein- und Kohlenhydratstoffwechsel hervor.

Aus der DE 195 21 642 sind Implantate bekannt, die zumindest teilweise aus einem resorbierbaren Material bestehen und die in diesem resorbierbaren Material einen antibiotischen Wirkstoff aufweisen, wobei dieser Wirkstoff während der gesamten Abbauphase des resorbierbaren Materials in die Umgebung abgegeben wird. Als antibiotischen Wirkstoff wird in dieser Patentschrift insbesondere Gentamycin genannt.

Nachteilig an der Verwendung von Gentamycin ist, dass dessen therapeutische und toxische Konzentration individuell stark variiert. So wurden im Zusammenhang mit Gentamycin Nebenwirkungen wie Nephrotoxizität, neuronale Blockaden und insbesondere vestibuläre und cochleäre Störungen beschrieben.

Weitere in diesem Zusammenhang nennenswerte Verbindungen sind die Cytostatika Taxol (Paclitaxel) und Rapamycin und deren Derivate. Jedoch sind auch hier aus der Literatur zahlreiche Nebenwirkungen und Komplikationen bei der Verwendung als Stent-Beschichtung bekannt, z.B. die starke Thrombogenität von Taxol (F. Liistro, A. Colombo, "Late acute thrombosis after paclitaxel eluting stent implantation", Heart, 86: 262-264 (2001)).

Bei der Verwendung von Rapamycin als Stent-Beschichtung (US 2001 027 340) zeigte sich in aktuellen klinischen Studien nur eine eingeschränkte Reduzierung der Restenose (SIRIUS-Studie der Firma Cordis: 10 % Restenose). Nachteilig bei diesen Cytostatika ist neben der hohen Toxizität die chemische Instabilität und die daraus resultierende Schwierigkeit der exakten Dosierung. So zeigten einige Patienten in der obengenannten Studie auch Symptome einer Überdosierung (Ausdünnung der Gefäßwand).

Ferner besteht z.B. bei den im Stand der Technik bekannten beschichteten Stents die Gefahr, dass bei Anpassungsvorgängen des Stents an das betreffende Gefäß - in Form von Ausdehnungen oder Kompressionen - das Beschichtungsmaterial beschädigt werden. Die Beschichtung und die Freisetzung der biologisch aktiven Substanzen werden dadurch in ihrer jeweiligen Funktion und Wirkung beeinträchtigt.

Weiterhin besteht ein großes Bedürfnis, die Abgabe der biologisch aktiven Substanz in einer Beschichtung an die Umgebung des Implantats einfacher und genauer kontrollieren zu können.

Aus der WO 01/64214 ist es bekannt, Topoisomeraseinhibitoren, wie bspw. Camptothecin, Anthracycline und 1,4-Naphthochinone wie Juglon, Menadion und Plumagin zur Behandlung von Entzündungskrankheiten einzusetzen.

Die WO 01/21326 offenbart ein Verfahren zur Herstellung eines Polymers mit einer Trägeroberfläche, wobei ein Beschichtungsmittel aufgetragen wird, welches ein zyklisches Diketon, wie bspw. Naphthochinon aufweisen kann.

Trotz der im Stand der Technik vielfältig diskutierten verschiedenen abbaubaren Beschichtungsmaterialien und trotz der Vielzahl von in diesem Zusammenhang untersuchten antibiotisch, antimikrobiell oder anti-koagulierend wirkenden Mitteln, sind Restenosen von Gefäßen und Abstoßungsreaktionen von Implantaten nach wie vor ein großes Problem. Demnach besteht trotz alledem ein großes Bedürfnis an Vorrichtungen, die eine dauerhafte Freihaltung von bspw. Gefäßen und ein gute Bioresorbierbarkeit ermöglichen.

Es ist daher Aufgabe der vorliegenden Erfindung, eine Beschichtung, die ein Polymer aufweist, mit einem neuen biokompatiblen biologisch aktiven Wirkstoff bereitzustellen, wobei vorzugsweise bei einer Optimierung der mechanischen Eigenschaften der Beschichtung bestimmte Mengen dieses biologisch aktiven Wirkstoffs eingelagert werden können und dessen Freisetzung aus der Beschichtung einfach kontrolliert werden kann.

Gemäß der vorliegenden Erfindung wird diese Aufgabe bei der eingangs genannten Beschichtungszusammensetzung dadurch gelöst, dass der biologisch aktive Wirkstoff Naphthazarin und/oder ein Naphthazarin-Derivat ist.

Die der Erfindung zugrunde liegende Aufgabe wird auf diese Weise vollkommen gelöst.

Der Erfinder hat erkannt, dass es durch die Verwendung von Polymeren und von bestimmten Naphthazarin-Derivaten in einer Beschichtung möglich ist, eine implantierbare medizinische Vorrichtung zu schaffen, die hervorragende mechanische Eigenschaften aufweist, d.h. sowohl Kompressionen als auch Expansionen standhalten kann, und dass die Einlagerung von Naphthazarin-Derivaten keinen negativen Einfluss auf die mechanischen Eigenschaften hat, vielmehr bleibt die Substanz als biologisch aktiver Wirkstoff erhalten.

Der Erfinder hat ferner erkannt, dass es durch den Einsatz von bestimmten Naphthazarin-Derivaten möglich ist, optisch zu kontrollieren, ob und in welchem Umfang die Beschichtung von implantierbaren medizinischen Vorrichtungen erfolgreich verlaufen ist. Dies wird durch die den Naphthazarin-Derivaten zugrunde liegende Farbigkeit erreicht: Enthält eine Beschichtung ein Naphthazarin-Derivat, so kann aufgrund der Einfärbung ermittelt werden, ob eine Vorrichtung unbeschichtet, beschichtet oder nur zum Teil beschichtet ist.

Naphthazarin ist im Stand der Technik als Grundkörper vieler Naturfarbstoffe bekannt, die darüber hinaus auch Heilmittel darstellen. Durch den Einsatz von Naphthazarin und/oder Naphthazarin-Derivaten können zwei vorteilhafte Wirkungen mit einem Wirkstoff erzielt werden. Zum einen sind diese Naturstoffe farbig, so dass der Erfolg der Beschichtung optisch kontrolliert werden kann, und andererseits weisen sie gleichzeitig eine heilende Wirkung auf, was den Zusatz weiterer biologisch aktiver Substanzen in die Beschichtung unnötig macht.

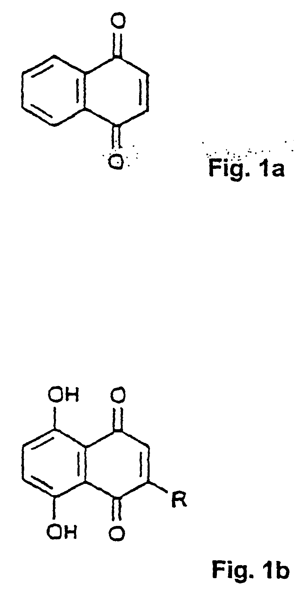

In Fig. 1b ist der Grundkörper von Naphthazarin gezeigt. Naphthazarin wiederum gilt als Derivat von 1,4-Naphthochinon, dessen Grundgerüst in Fig. 1a gezeigt ist.

Zu den Naphthazarin-Derivaten zählen vorliegend alle Verbindungen, die das Grundgerüst von Naphthazarin aufweisen, wobei der Rest R bspw. irgendein aliphatischer Rest sein kann, welcher acyclisch oder cyclisch, unverzweigt oder verzweigt, substituiert (bspw. Hydroxy-substituiert) vorliegen kann.

In einer weiteren Ausführungsform ist das Derivat aus der Gruppe umfassend Shikonin, Alkannin, Arnebin und Derivate davon ausgewählt. Besonders bevorzugt ist hierbei Shikonin.

Der Erfinder konnte in eigenen Versuchen zeigen, dass bei mit dem Naphthazarin-Derivat Shikonin beschichteten Stents eine Blutplättchen- und Fibroblasten-Aggregation verhindert werden konnte.

Das Wirkungsspektrum von Shikonin ist im Vergleich zu den im Zusammenhang mit Beschichtungen bereits verwendeten Verbindungen wie bspw. Rapamycin und Taxol wesentlich breiter. Shikonin, Alkannin und Derivate davon sind bereits seit langem als rote Naturfarbstoffe und als Heilmittel bekannt. So stellen bspw. Papageorgiou et al., "Chemie und Biologie von Alkannin, Shikonin und verwandten Naphthazarin-Naturstoffen", Angew. Chemie 111: 280-311, 1999) in ihrem Übersichtsartikel die seit längerem bekannten biologischen und pharmakologischen Eigenschaften dieser Wirkstoffe vor und behandeln bioorganische, präparative und medizinische Aspekte: So werden in dem Artikel andere Veröffentlichungen zitiert, in denen gezeigt wurde, dass Shikonin selber eine entzündungshemmende und antimikrobielle wirkung aufweist. In dem Artikel von Papageorgiou et al. wird ferner eine Veröffentlichung zitiert, in der eine antithrombotische Wirkung von wenigen Naphthazarin-Derivaten beschrieben ist, diese Wirkung konnte aber nicht eindeutig der Shikonin-Familie zugewiesen werden.

Shikonin besitzt erwiesenermaßen eine Antitumorwirkung, ist antimykotisch, antimikrobiell und wundheilend. Die bisher verwendeten Cytostatika (bspw. Rapamycin oder Taxol) erreichen das einzigartige Wirkungsprofil von Shikonin nicht.

Hisa et al., "Shikonin, an Ingredient of Lithospermum Erythrorhizon, Inhibits Angiogenesis *in Vivo* and *in Vitro*", Cancer Res. 18: 783-790, 1998, zeigten, dass Shikonin die Proliferation von bovinen Endothelzellen inhibieren konnte, was zu einer Inhibierung der Angiogenese führte. Hingegen ist in diesem Artikel nicht beschrieben oder dargelegt, ob und in welcher Weise Shikonin oder mit Shikonin verwandte Substanzen dazu in der Lage sind, die Proliferation von Fibroblasten, insbesondere von Myofibroblasten, deren Proliferation wie zuvor erwähnt an der Restenose von Gefäßen beteiligt ist, zu verhindern.

Insbesondere unter Berücksichtigung des Artikels von Sakaguchi et al., "Granulomatous Tissue Formation of Shikon and Shikonin by Air Pouch Method", Biol. Pharm. Bull 24(6): 650-655, 2001, in dem dargelegt ist, dass Shikonin die Gefäßneubildung stimuliert, waren die von dem Erfinder gezeigten Ergebnisse nicht zu erwarten. Im Gegenteil, die bekannten Eigenschaften von Shikonin sprachen bisher gegen eine Verwendung von Shikonin oder anderer Naphthochinon- oder Naphthazarin-Derivate im Zusammenhang mit implantierbaren medizinischen Vorrichtungen. Erst der Erfinder konnte zeigen, dass Shikonin die Aggregation von Blutplättchen und damit sowohl Thrombosen als auch Langzeit-Restenosen verhindern kann.

Zur Beschichtung von implantierbaren medizinischen Vorrichtungen wurden Naphthazarine bisher nicht vorgeschlagen.

Darüber hinaus sind Naphthazarin-Derivate dazu geeignet, die Freisetzungskinetik in der Entwicklung besser zu überprüfen. Andere Farbstoffe, bspw. ohne jegliche biologische Aktivität, wären lediglich weitere Fremdstoffe in der Beschichtung und würden dadurch die Gefahr einer allergischen oder entzündlichen Reaktion erhöhen. Demnach wird durch die Verwendung von Shikonin bzw. von anderen Naphthazarin-Derivaten eine optische Endkontrolle bereitgestellt, ob und wie gut ein Implantat beschichtet worden ist.

Dabei ist nicht ausgeschlossen, dass die Heschichtungszusaiamensetzung auch mehrere Naphthazarin-Derivate aufweist oder aber in die Beschichtungszusammensetzung weitere Begleitstoffe mit eingearbeitet werden, wie bspw. antikoagulierende, antimikrobielle oder antiinflammatorische Substanzen.

In einer weiteren bevorzugten Ausführungsform enthält die Beschichtungszusammensetzung Naphthazarin und/oder ein Naphthazarin-Derivat in einem Anteil von 0,01 bis 1 Gew.-%.

Ferner ist bevorzugt, wenn Napathazarin und/oder Naphthazarin-Derivat in einem Anteil enthalten ist, der ausgewählt ist aus der Gruppe umfassend 0,04 Gew.-%, 0,05 Gew.-%, 0,06 Gew.-%, 0,07 Gew.-%, 0,08 Gew.-%, 0,09 Gew.-%, 1 Gew.-%.

Der Erfinder konnte in eigenen Versuchen zeigen, dass die Verwendung von 0,1 bis 1 Gew.-% an einem Naphthazarin-Derivat, insbesondere Shikonin, in der Beschichtung geeignet ist, um eine ausreichende inhibierende Wirkung auf die Blutplättchen und Fibroblasten-Adhäsion auszuüben.

Das Polymer kann dabei ein biokompatibles Polymer sein, aus dem der biologisch aktive Wirkstoff herausdiffundiert, oder ein resorbierbares Polymer, aus dem der Wirkstoff durch Abbau des Polymers freigesetzt wird.

In einer weiteren Ausführungsform ist bevorzugt, wenn das Polymer ein resorbierbarer Polyester ist und insbesondere aus der Gruppe umfassend Polyglykolsäure, Polymilchsäure, Polycaprolakton, Polyhydroxyalkanoate und Copolyester davon ausgewählt ist.

Derartige resorbierbare Polyester und Copolyester sind im Stand der Technik hinreichend bekannt und haben sich insbesondere in medizinischen Verwendungen hinreichend bewährt.

Hierbei ist insbesondere bevorzugt, wenn das Polyhydroxyalkanoat aus der Gruppe umfassend Polyhydroxybutyrat, Polyhydroxyvalerat und Copolyester davon ausgewählt ist.

Der Erfinder konnte in eigenen Versuchen zeigen, dass insbesondere Copolyester aus Polyhydroxybutyrat und Polyhydroxyvalerat als Beschichtungsmaterial hervorragende Eigenschaften besitzen. Durch diese Polyester wird gewährleistet, dass die Beschichtung sowohl biologisch abbaubar ist als auch ausgezeichnete mechanische Eigenschaften aufweist. Ferner zeigte sich in eigenen Versuchen, dass ein in dieses Copolyester eingelagertes Naphthazarin-Derivat ausgezeichnete biologische Aktivitäten aufwies.

Es ist bekannt, dass zahlreiche organische, biologisch abbaubare Polymere in der Lage sind, Wirkstoffe innerhalb eines gewissen Zeitfensters freizusetzen. Viele Materialien sind aber als Beschichtung nicht geeignet, da sie nicht vollständig biokompatibel sind. So zeigten bspw. van der Giessen et al., "Marked Inflammatory Sequelae to Implantation of Biodegradable and Nonbiodegradable Polymers in Porcine Coronary Arteries", Circulation 94:1690-1697, 1996, dass Polylactide und andere vermeintlich als biokompatibel geltende Polymere beim Abbau im Körper zu entzündlichen Gewebe-Reaktionen führten.

Weiterhin ist bekannt, dass viele biologisch abbaubare Polymere nicht die entsprechenden mechanischen Eigenschaften aufweisen. So können bspw. kristalline Bereiche zu einer plötzlichen Rissbildung führen. Aus diesen Gründen muss eine Beschichtung für eine implantierbare medizinische Vorrichtung besondere mechanische Eigenschaften aufweisen, und sowohl Kompressionen als auch Expansionen - bspw. bei Inflation des Katheters - standhalten.

In einer weiteren Ausführungsform ist in dem Copolyester Polyhydroxyvalerat in einem Anteil von 20 bis 30 Gew.-%, vorzugsweise von 25 Gew.-%, und Polyhydroxybutyrat in einem Anteil von 70 bis 80 Gew.-%, vorzugsweise von 75 Gew.-%, enthalten.

In eigenen Versuchen konnte der Erfinder zeigen, dass dieses Verhältnis für einen Einsatz als Beschichtungsmaterial besonders geeignet ist, da mit diesem Verhältnis eine gute Löslichkeit gewährleistet wird und gleichzeitig die hervorragenden mechanischen Eigenschaften des Polyesters erzielt werden.

Ferner ist in einer weiteren Ausführungsform bevorzugt, wenn das resorbierbare Polymer und das Shikonin in zumindest einem Lösungsmittel, vorzugsweise Dimethylacetamid und/oder Tetrahydrofuran gelöst vorliegen.

Es hat sich gezeigt, dass durch Lösen der wirksamen Komponenten der Beschichtung in diesen Lösungsmitteln deren mechanische bzw. biologische Eigenschaften nicht beeinträchtigt werden.

Die Erfindung betrifft weiterhin ein Verfahren zur Beschichtung einer implantierbaren medizinischen Vorrichtung mit den Schritten:

(a) Aufbringen der neuen Beschichtungszusammensetzung auf die implantierbare medizinische Vorrichtung,

(b) Trocknen der implantierbaren medizinischen Vorrichtung und

(c) ggf. Wiederholen der Schritte (a) und (b).

In einer Ausführungsform ist hierbei bevorzugt, wenn die Beschichtungszusammensetzung auf die implantierbare medizinische Vorrichtung aufgesprüht wird.

Zum Aufsprühen können verschiedene Techniken angewendet werden, die allesamt im Stand der Technik hinreichend bekannt sind.

In einer anderen bevorzugten Ausführungsform wird die Beschichtung durch Eintauchen der implantierbaren medizinischen Vorrichtung in die Beschichtung aufgebracht.

Die Beschichtungsvorgänge werden dabei so oft wiederholt, bis die erwünschte Schichtdicke für die Beschichtung auf der implantierbaren medizinischen Vorrichtung erreicht ist, bspw. eine Schichtdicke von 1 bis 100 $\mu$m.

Eine bevorzugte Ausführungsform des erfindungsgemäßen Verfahrens zur Beschichtung einer implantierbaren medizinischen Vorrichtung besteht darin, dass eine Beschichtung aufgebracht wird, die ein Polyhydroxybutyrat-Polyhydroxyvalerat-Copolyester aufweist, in dem das Verhältnis Polyhydroxybutyrat : Polyhydroxyvalerat 3:1 ist, und in dem Shikonin in einem Anteil von 0,01 bis 5 Gew.-% enthalten ist.

Der Erfinder hat erkannt, dass mit dieser Zusammensetzung eine besonders geeignete Beschichtung geschaffen werden kann, mit welcher neben den optimalen mechanischen Eigenschaften einer Beschichtung auch die Doppelfunktion von Shikonin - als Farbstoff und biologisch aktiver Wirkstoff - wirkungsvoll genutzt werden kann. Die Eigenschaften von Shikonin - Farbstoff und Wirkstoff - bleiben auch nach Einbau in die erfindungsgemäße Beschichtung erhalten.

In einer weiteren bevorzugten Ausführungsform wird bei dem Verfahren zur Beschichtung einer implantierbaren medizinischen Vorrichtung ein Stent als Vorrichtung eingesetzt.

Hierbei können bspw. Stents eingesetzt werden, die zumindest ein Metall und/oder ein Kunststoffmaterial aufweisen. Derartige Stents und Verfahren zur Beschichtung von derartigen Stents sind im Stand der Technik hinreichen bekannt.

Es ist aber nicht ausgeschlossen, dass auch andere Formen von implantierbaren medizinischen Vorrichtungen mit der erfindungsgemäßen Beschichtungszusammensetzung beschichtet werden können. So kommen bspw. auch Hautimplantate, ein Knorpel- oder Knochenersatz in Frage, die eben, rechteckig, zylindrisch oder als Klappe ausgebildet sein können.

Falls als implantierbare medizinische Vorrichtung eine Gefäßprothese verwendet wird, so kann deren tubuläre Ausgestaltung beliebig geformt sein, also bspw. als verzweigter oder unverzweigter Tubus etc.

Die Erfindung betrifft ferner die Verwendung einer wie oben angegebenen neuen Beschichtungszusammensetzung zur Beschichtung von implantierbaren medizinischen Vorrichtungen.

Die Erfindung betrifft ferner die Verwendung von Naphthazarin und/oder Naphthazarin-Derivaten, insbesondere von Shikonin, zur Herstellung einer Beschichtungszusammensetzung für eine implantierbare medizinische Vorrichtung und die Verwendung zur Beschichtung einer implantierbaren medizinischen Vorrichtung.

Die Erfindung betrifft ferner eine implantierbare medizinische Vorrichtung, die mit einer wie oben genannten neuen Beschichtungszusammensetzung beschichtet ist, und insbesondere Stents.

Weitere Vorteile ergeben sich aus der nachstehenden Beschreibung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen und durch die Figuren näher erläutert. Es zeigen

Fig. 1a    die Formel der Substanz 1,4-Naphthochinon;

Fig. 1b    die allgemeine Formel des Grundkörpers von Naphthazarin-Derivaten.

## **Beispiel**

## **1. Verwendete Beschichtungszusammensetzungen**

Folgende Polymer-Zusammensetzungen wurden als Beschichtungsmaterial getestet:

- eine Komposit-Beschichtung aus dem nicht resorbierbaren Polyurethan Elastollan, mit und ohne Shikonin;

- ein biologisch abbaubarer Copolyester aus Poly($\beta$-hydroxybutyrat-co-$\beta$-hydroxyvalerat) (im folgenden P(HB-HV)) mit einem Anteil von 12 Gew.-% Polyhydroxyvalerat;

- ein biologisch abbaubarer Copolyester aus P(HB-HV) mit einem Anteil von 25 Gew.-% Polyhydroxyvalerat, mit und ohne Shikonin.

Als Lösungsmittel wurden Dimethylacetamid (im folgenden DMAA) und Tetrahydrofuran (im folgenden THF) getestet.

Dabei erwies sich eine Zusammensetzung von 25 Gew.-% Polyhydroxyvalerat (im folgenden PHV) im Verhältnis zu 75 % Polyhydroxybutyrat (im folgenden PHB) am geeignetsten. Darüber hinaus konnte eine Zusammensetzung mit diesem Verhältnis mit einer maximalen Konzentration von bis zu 1,5 Gew.-% in dem jeweiligen Lösungsmittel unter Erhitzen in beiden Lösungsmitteln gelöst werden.

Diesbezüglich wurde eine Arbeitslösung (I) enthaltend P(HB-HV) mit 25 Gew.-% PHV mit folgender Zusammensetzung für die Beschichtungszusammensetzung verwendet:

- P(HB-HV): 1,5 g
- DMAA oder THF: 100 ml

Neben diesem reinen Copolyester wurde eine weitere zusammengesetzte Beschichtung getestet, nämlich P(HB-HV) mit einem Gehalt von 25 Gew.-% PHV und mit Shikonin. Die hierzu verwendete Arbeitslösung (II) wies die folgende Zusammensetzung auf:

- P(HB-HV): 1,5 g

- Shikonin: von 15 bis 75 mg
- DMAA oder THF: 100 ml.

Für die Durchführung der Tests wurden zum einen mit den Arbeitslösungen I und II beschichtete Stents (aus rostfreiem Stahl mit elektropolierter Oberfläche, Translumina GmbH, Hechingen, Deutschland) verwendet und zum anderen in Petrischalen gegossene Polymerfilme aus den angegebenen Arbeitslösungen I und II.

## 2. Zell-Adhäsions- und Zell-Proliferationsstudien

a) Elastollan-Filme

Material und Methoden

Getestet wurden 0,1 und 0,5 Gew.-% Shikonin enthaltende Elastollan Filme und Elastollan-Filme ohne Shikonin.

Obgleich Elastollan ein nicht-resorbierbares Polymer darstellt, so zeigen die mit diesem Polymer erzielten Ergebnisse doch die Wirksamkeit von Shikonin als Inhibitor der Zelladhäsion und -proliferation. Dies bedeutet, dass hier der Wirkstoff durch Auswaschung und nicht durch Abbau der Elastollan-Matrix freigesetzt wird.

Bei diesen Versuchen wurden humane embryonale Fibroblasten eingesetzt. Die Zellen wurden in Eagle's Medium unter Zusatz von 10 % fetalem Kälberserum kultiviert und zweimal in einer Woche unter Verwendung einer Trypsin-EDTA-Lösung passagiert. Die nach der 14. Passage vorliegenden Zellen wurden für die Tests verwendet. Als Negativkontrolle wurde in Kulturmedium gelöstes Mitomycin C in einer Konzentration von 20 $\mu$g/ml verwendet.

Getestet wurden Extrakte aus Elastollan-Filmen ohne Shikonin, Elastollan-Filme mit 0,1 Gew.-%, 0,5 Gew.-%, 1 Gew.-% und 5 Gew.-% Shikonin (bezogen auf Polyurethan), sowie Shikonin alleine.

Zur Gewinnung der Extrakte wurden Schälchen, ausgekleidet mit den zu testenden Polymeren (also Elastollan-Filme ohne bzw. mit Shikonin), 3 h bei 37 °C mit Eagle's Medium inkubiert. Ungelöste Feststoffe wurden durch Filtern entfernt. Die Kontrolle mit lediglich Shikonin (in Kultur-Medium) wurde unter den gleichen Bedingungen kultiviert.

Die jeweiligen Filtrate wurden als Test-Extrakte eingesetzt, wobei der pH-Wert der Shikonin-enthaltenden Elastollan-Extrakte zuvor mit 0,1 N HCl eingestellt wurde, und wobei dem Kontroll-Extrakt (Elastollan ohne Shikonin) und dem Extrakt mit Shikonin alleine die gleiche Menge an Phosphat-Puffer hinzugefügt wurde, die zur Einstellung des pH-Wertes der Elastollan/Shikonin-Extrakte notwendig war.

Parallel dazu wurden jeweils 1 ml der Zell-Suspension (Fibroblasten, siehe oben) mit 4 x 10$^4$ Zellen/ml auf 24-wellPlatten aufgebracht. Nach einer 24-stundigen Kultivierungszeit wurde das Medium entfernt und die zu testenden Extrakte hinzugefügt (Positivkontrolle: frisches Medium ohne Extrakt; Negativkontrollen: Zugabe von Mitomycin C für 2 h).

Anschließend wurden die Zellen mit Hanks-Lösung gewaschen und in die Wells frisches Medium gegeben. Anschließend wurden die Zellen über 72 h inkubiert. Nach dieser Inkubationszeit wurden die Kulturen zweimal mit Phosphatpuffer gewaschen und mit 2,5% Glutaraldehyd für 30 min bei 4°C inkubiert. Anschließend wurden sie wiederum zweimal gewaschen und unter Luftfeuchtigkeit für 3 h bei 37°C mit Giemsa gefärbt.

Die von den Zellen zurückgehaltene Farbe wurde mit einer Phosphat-Puffer/Alkohol-Mischung (1:1) über 15 min bei Raumtemperatur eluiert. Die optische Dichte der erhaltenen Lösungen wurde durch ein Spektrophotometer mit einer Wellenlänge von 620 nm bestimmt.

Ergebnisse

In der Positivkontrolle (getesteter Extrakt: lediglich Kulturmedium) bedeckten die Fibroblasten nahezu die gesamte Oberfläche des Wells, wobei sie eine längliche Form und ein typisches Wachstums-Muster zeigten.

In der Negativkontrolle (getesteter Extrakt: Kulturmedium + Mitomycin C) konnte, kein Zellwachstum beobachtet werden. Die Zelldichte war gering und entsprach der vom zweiten Tag des Versuchs. Die Zellen besaßen eine längliche Form, standen jedoch nicht in Kontakt miteinander. Einige Zellen waren abgerundet oder waren lysiert.

Das Zellwachstum auf Extrakt aus Elastollan allein war ähnlich wie in der Positiv-Kontrolle. Bei Verwendung von Shikonin-enthaltenden Elastollan-Extrakten konnte eine inhibitorische und sogar cytotoxische Wirkung beobachtet werden: Einzelne Zellen waren angeheftet, die meisten waren abgerundet.

Dieser Test zeigte, dass Shikonin und Extrakte aus Shikonin-enthaltenden Elastollan-Filmen in vitro einen inhibitorischen Effekt auf menschliche Zellen besitzen.

b) *In vitro*-Adhäsion von humanen embryonalen Fibroblasten der Lunge auf Elastollan- und Shikonin-enthaltenden Elastollan-Filmen

Zu diesem Test wurden Petrischalen, beschichtet mit Filmen aus Elastollan- und Shikonin-enthaltenden Elastollan-DMAA-Lösungen, verwendet. Die Konzentration von Shikonin in den Polyurethanproben betrug 0,01 Gew.-%, 0,05 Gew.-% und 0,5 Gew.-% (bezogen auf Polyurethan). Eine Petrischale aus Plastik diente als Positivkontrolle.

Die Zellen wurden auf die Oberfläche der mit den Polymer-Zusammensetzungen beschichteten Petrischalen mit einer Konzentration von 4 x $10^4$ Zellen/ml in Eagle's Medium mit 10 % fetalem Kälberserum ausgesät. Die Anzahl der angehefteten Fibroblasten wurde nach einer Inkubation von 0,5 und 2 h bei 37°C ausgezählt.

Ergebnisse

Die erhaltenen Auszählungen sind in der nachfolgenden Tabelle I dargestellt.

Wie aus der Tabelle durch den Vergleich mit der Positivkontrolle ersichtlich ist, inhibiert eine Konzentration von 0,5 Gew.-% Shikonin in den Elastollan-Filmen die Anheftung von Fibroblasten praktisch vollständig:

| Art der Oberfläche | Anzahl der ausgezählten Fibroblasten (nach Inkubationszeit von) | |
| --- | --- | --- |
| | 0,5 h | 2 h |
| Kontrolle (Plastik-Petrischale) | ca. 60 | ca. 95 |
| Elastollan | 10 - 40 | keine |
| Elastollan + Shikonin (0,01 Gew.-%) | < 20 | keine |
| Elastollan + Shikonin (0,05 Gew.-%) | ca. 30 | keine |
| Elastollan + Shikonin (0,5 Gew.-%) | 0 - 5 | keine |

c) Adhäsion von Blutplättchen auf Copolyester P(HB-HV)-Filmen und auf Shikonin-enthaltenden Copolyester P(HB-HV) -Filmen P(HB-HV)-Sh) und auf mit diesen Filmen beschichteten Stents

Es ist bekannt, dass die Adhäsion von Blutplättchen auf Biomaterialien die Blutkompatibilität von medizinischen Vorrichtungen mit Bezug auf die Blut- und Gewebezellaktivierung wiedergibt.

Man vermutet, dass die Adhäsion in mehreren Schritten verläuft: Zunächst binden die Blutplättchen an die Oberfläche, werden anschließend aktiviert und entwickeln Pseudopodien, anschließend breiten sie sich aus und bilden Aggregate. Die darauf folgende Freisetzung von intrazellulären Komponenten, einschließlich der Blutverklumpungsfaktoren, stimuliert die Adhäsion und die Aggregation von weiteren Blutplättchen.

Daneben stellt die Infiltration von aktiv proliferierenden Myocyten aus dem Medium in die Intima, begleitet durch die Produktion von überschüssigen extrazellulären Matrixkomponenten (Kollagen, Proteo-Aminoglykane), den vermuteten grundlegenden Mechanismus der Restenose dar. Die sofortige Blutplättchenablagerung an der Stelle, an der das Gefäß verletzt wurde, und die die nachfolgende Freisetzung von myoproliferativen Substanzen (bspw. der Wachstumsfaktor (PDGF), β-transformierender Faktor (β-TGF), "endothelium-derived growth factor (EDGF)", etc. stellen wahrscheinlich die stimulierenden Faktoren dar.

Aus diesen Gründen spielt die Adhäsion von Blutplättchen während einer Stent-Implantation sowohl bezüglich von Thrombosen als auch von Restenosen eine Schlüsselrolle.

Der Aktivierungsstatus von adhärierenden Blutplättchen kann durch deren Morphologie eingeschätzt werden. Je größer die Auswirkung des Materials auf die Blutplättchen ist, desto mehr adhärierende Zellen sind auf dem Material verteilt oder aggregiert.

Für diesen Test wurden sowohl unbeschichtete Stents als auch mit P(HB-HV)-Sh beschichtete Stents getestet. Die durchschnittliche Schichtdicke der Beschichtung betrug ungefähr 15 bis 20 $\mu$m.

Folgende Lösungen dienten zur Beschichtung der Stents:

- 0,75 % P(HB-HV) mit 25 % HV in DMAA oder THF;
- 0,75 % P(HB-HV) mit 25 % HV + 0,5 Gew.-% Shikonin (bezüglich des Gewichts von P(HB-HV) in DMAA oder THF.
- 0,75 % P(HB-KV) mit 25 % HV + 1 Gew.-% Shikonin (bezüglich des Gewichts von P(HB-HV) in DMAA oder THF.

Zur Beschichtung wurden die Stents mehrmals in die verschiedenen Lösungen eingetaucht oder besprüht. Die Trocknungszeit zwischen den einzelnen Sprühvorgängen betrug zwischen 2 und 15 min. Abschließend wurden die Stents für 30 min bei 45 bis 50 °C getrocknet. Die Gesamtmenge an Shikonin auf den besprühten P(EB-KV)-1,0Sh-Stents betrug 2 $\mu$g bis 4 $\mu$g.

Die Shikonin-Beschichtung der Stents erwies sich in expandiertem Zustand über einen Zeitraum von 28 Tagen als stabil.

Des weiteren wurden Polymerfilme aus den angegebenen Lösungen (P(HB-HV) mit oder ohne Shikonin) auf Platten aus rostfreiem Stahl aus den gleichen Lösungen mit einer Schichtdicke von 20 bis 30 $\mu$m getestet. Als Kontrolle diente die Oberfläche eines unbeschichteten Stents.

Vorbereitung des Bluts

Gesamtblut von gesunden, erwachsenen Spendern wurde in silikonisierte Glasgefäße gegeben. Durch Zugabe von Natriumcitrat in einem Verhältnis von (Blut : Natriumcitrat) 9:1 wurde die Blutgerinnung von 10 ml Blut verhindert. Blutplättchen-angereichertes Plasma konnte durch Zentrifugieren des Gesamtbluts bei 100 x g für 20 Minuten bei Raumtemperatur gewonnen werden. Die Blutplättchen-angereicherte Plasmafraktion wurde mit einer Plastikpipettenspitze abgenommen und sofort in den Versuchen eingesetzt.

50 $\mu$l-Tropfen dieser Blutplättchen-angereicherten Plasmafraktion wurden auf die Oberflächen der Platten-Proben gegeben und für 15 min inkubiert. Zum Test der beschichteten Stents wurden diese in ein Gefäß mit Blutplättchen-angereichertem Plasma gegeben und für 30 Minuten inkubiert. Die Anzahl der Blutplättchen, die während dieses Zeitraums an die Oberfläche adhärierten, war ausreichend für eine qualitative Analyse, da die Plättchen keine großen Thrombus-ähnlichen Strukturen bildeten. Die Proben wurden mit physiologischer Kochsalzlösung gewaschen, um nicht-adsorbierende Plasmaproteine und schwach adhärierende Blutplättchen abzuwaschen. Anschließend wurden die Proben in 2,5 %igem Glutaraldehyd fixiert, und danach nach üblichen Verfahren mit steigendem Ethanol-Gehalt dehydriert.

Die Adhäsion der Blutplättchen wurde durch Raster-ElektronenMikroskopie (SEM) untersucht (JSM T330, JEOL, Japan). Durch Beschichtung von Kupfer mit 1,2 kV, 10 mA für 7 Minuten (JEOL JFC-1100, Japan) wurden alle Proben leitfähig gemacht. Die mikroskopischen Untersuchungen wurden mit einer Spannung von 5 kV durchgeführt. Für jede Probe wurden 25 Abschnitte mit einer Größe von jeweils 400 $\mu$m$^2$ zufällig ausgewählt. Anschließend wurde die qualitative Gesamtblütplättchenanzahl $N_{ges}$ und die Anzahl der Blutplättchen $N_l$ der folgenden zwei Kategorien von Zellen ausgewertet, die explizit die Aktivierung der Blutplättchen wiedergeben. Jede Kategorie enthält zwei morphologische Klassen an adhärierenden Blutplättchen:

Ia) einzelne, nicht-aktivierte oder nur leicht aktivierte deformierte Zellen;
Ib) Pseudopodien-ausbildende Zellen oder Zellen in einem frühen Stadium des Ausbreitens.

IIa) Voll ausgebreitete Blutplättchen;
IIb) Aggregate (aus zwei oder mehr Blutplättchen).

Mit Bezug auf diese Klassifizierung interagieren Blutplättchen aus der Kategorie I nur schwach mit der Oberfläche; im Gegensatz hierzu findet zwischen der Oberfläche und den Blutplättchen der Kategorie II eine starke Wechselwirkung statt.

Ergebnisse

a) Beschichtung mit einer 0,75 Gew.-%igen Lösung aus P(HB-HV) und P(HB-HV)+Shikonin in DMAA

Die Anzahl der Zellen auf den unbeschichteten Stents erwies sich als sehr gering, dort, wo adhärierende Blutplättchen vorlagen, waren praktisch alle Zellen, komplett auf dem unbeschichteten Stent ausgebreitet. Die Gesamtzahl auf dem P(HB-HV)-Film war höher als die für die Kontrolle und alle morphologischen Klassen der Zellen waren auf dieser Oberfläche vorhanden, die Anzahl der Aggregate erwies sich jedoch als sehr niedrig.

Shikonin hingegen konnte die Menge an adhärierenden Blutplättchen im Vergleich zu lediglich mit P(HB-HV) beschichteten Filmen deutlich reduzieren. Hier konnten nur zwei morphologische Klassen der Zellen der Kategorie I beobachtet werden: leicht aktivierte Blutplättchen und Pseudopodien-bildende Zellen.

Ausgehend von diesen Ergebnissen ergibt sich, dass P(HB-HV)-Sh-Filme geeigneter sind als Oberflächen mit reinem P(HB-HV), da die Shikonin-haltigen Oberflächen eine geringere Affinität zur Zellbindung aufwiesen.

Um den Zusammenhang zwischen Blutplättchenaktivierung und Shikoninkonzentration zu bestimmen, wurde die Blutplättchenadhäsion auf Filmen aus reinem P(HB-HV), aus P(HB-HV) mit 0,1 Gew.-% Shikonin (P(HB-HV)-0,1Sh) und aus P(HB-HV) mit 0,5 Gew.-% Shikonin (P(HB-HV)-0,5Sh) für eine Inkubationszeit von 15 Minuten und 30 Minuten bestimmt. Hierdurch konnte nachgewiesen werden, dass der Zusatz von Shikonin die Anzahl und den Grad der Aktivierung der Blutplättchen reduzieren konnte.

b) Beschichtung mit einer 0,75 Gew.-%igen Lösung aus P(HB-HV) und P(HB-HV)+Shikonin in THF

Die Beschichtungen wurden als Multilayer-Filme auf expandierten und nicht-expandierten Stents aufgetragen. Die Stents wurden mit dem Polymer durch Eintauchen dieser in die verdünnte Arbeits-Polymer-Lösung beschichtet (ca. 2 bis 4 Mal). Jede Schicht wurde anschließend mit heißer Luft getrocknet.

Eine vergleichende Analyse der Adhäsion von Blutplättchen auf dem unbeschichteten Stent, auf einem mit diamantähnlichem Kohlenstoff-beschichteten Stent und auf einem mit P(HB-HV)-0,5Sh-beschichteten Stent wurde durchgeführt. Die Inkubationszeit der Proben in mit Blutplättchen angereichertem Plasma betrug von 15 bis ca. 30 Minuten.

Auf den mit P(HB-HV)-0,5Sh-beschichteten Stents wurden keine Blutplättchen beobachtet. In der gleichen Zeit konnten jedoch auf den unbeschichteten und auf den mit Kohlenstoff beschichteten Stents Blutplättchen gefunden werden. Dies bedeutet, dass die Eigenschaften von Shikonin bezüglich der Aktivierung der Blutplättchen auch bei einer Verwendung von THF als Lösungsmittel erhalten bleiben.

c) Beschichtung mit 1 Gew.-% Shikonin (P(HB-BV-1,0Sh) im Vergleich zu diamant-ähnlich beschichteten (DLC) Stents

Bei der vergleichenden Analyse der mit 1 Gew.-% Shikonin beschichteten Stents mit DLC-beschichteten Stents, zeigte sich, dass bei den P(HB-HV)-1,0Sh beschichteten Stents *in vitro* praktisch keine Plättchen an den beschichteten Stents adhärierten. Andererseits waren auf den DLC-beschichteten Stents einzelne ausgebreitete Plättchen aufzufinden.

## 3. Bestimmung der biologischen Eigenschaften der hergestellten Filme

Diese Untersuchungen wurden gemäß dem internationalen Standard für biologische Untersuchungen von medizinischen Vorrichtungen ISO 10993 und gemäß dem nationalen Standard GOST R ISO 10993 durchgeführt.

a) Bestimmung der hämolytischen Eigenschaften

Zur Bestimmung der Hämolyse wurden Extrakte der Materialien vorbereitet, und zwar von P(HB-HV) und von P(HB-KV)-0,5Sh, jeweils in physiologischer Kochsalzlösung. Zu diesen Extrakten wurde Gesamtblut hinzugegeben, anschließend erfolgte eine Inkubation für eine Stunde bei 37°C. Die Extrakte wurden entfernt, die Mischung zentrifugiert (50 Minuten bei 400xg) und der zellfreie Überstand sorgfältig entfernt. Für diesen Überstand wurde die Hämoglobinkonzentration photometrisch bestimmt und der hämolytische Index (%) durch das Verhältnis von freigesetztem Hämoglobin zu vorliegendem Hämoglobin berechnet. Als Kontrolle diente hierbei eine reine Kochsalzlösung.

Unter den gegebenen Bedingungen erwiesen sich beide Extrakte, also sowohl der von P(HB-HV) als auch der von P(HB-HV)-Sh als nicht hämolytisch (Hämolytischer Index: 0%).

b) Bestimmung der Aktivierung des Komplement-Systems

Um die Blutkompatibilität der Polymer-Filme zu testen, wurde die hämolytische Aktivität des Komplement-Systems des menschlichen Blutplasmas vor und nach dessen Inkubierung mit einer Filmprobe oder -extrakt bestimmt. Die getesteten Proben waren Filme aus P(HB-HV), P(HB-HV)-Sh und aus Cuprophan (Kontrolle).

Die Konzentration des Hämoglobins, welches durch die lysierende Wirkung des Komplement-Systems aus mit Antikörpern beschichteten Schaf-Erythrozyten freigesetzt wurde, gibt die Aktivität des Komplements in der Serum-Probe wieder. Der Parameter für das Assay war die Zeit, welche von 100 $\mu$l des getesteten Serums benötigt wird, um 50% von 5 ml sensibilisierte Schaf-Erythrozyten ($5 \times 10^8$ Zellen/ml) zu lysieren ($\tau_{1/2}$). Mittels Thermostaten wurde die Temperatur der Reaktionsansätze konstant auf 37°C gehalten.

Menschliches Serum wurde zuerst mit einem Puffer (enthaltend 0,15 mM $CaCl_2$ und 0,5 mM $MgCl_2$) verdünnt und anschließend mit oder ohne Polymer-Proben für 60 min bei 37°C inkubiert. Die Werte der restlichen Komplement-Aktivität $((CH_{50})^R\%)$ wurden als Kriterium für die aktivierenden Eigenschaften der Polymere folgendermaßen bestimmt:

$$(CH_{50})^R_i = (\tau_{1/2})_0 \ / \ (\tau_{1/2})(t)_{i(c)} \ 100\%$$

mit:

$(\tau_{1/2})_0$ = Zeit für eine 50% Lyse der Schaf-Erythrocyten durch das Serum-Komplement vor Inkubation mit der Probe;

$(\tau_{1/2})$ $(t)_i$ = Zeit für eine 50% Lyse der Schaf-Erythrocyten durch das Serum-Komplement nach der Inkubationszeit t mit der Polymer-Probe;

$(\tau_{1/2})$ $(t)_{(c)}$ = Zeit für eine 50% Lyse der Schaf-Erythrocyten durch das Serum-Komplement nach der Inkubationszeit t in der Kontrolle (ohne Polymer-Probe).

Die Ergebnisse dieser Tests sind in der folgenden Tabelle II zusammengefasst:

| Getestete Proben | $CH_{50}$ (n = 3) |
|---|---|
| Cuprophan (Kontrollmaterial) | $89 \pm 5$ |
| P(HB-HV)-Extrakt | $85 \pm 5 / 80 \pm 5$ |
| P(HB-HV)-Sh-Extrakt | $90 \pm 5 / 85 \pm 5$ |
| Serum nach 1 h Inkubation (Kontrollserum) | $98 \pm 5$ |

Wie aus der Tabelle ersichtlich ist, erwies sich die Komplement-Aktivität von mit P(HB-HV)-Sh-Extrakt inkubiertem Serum als niedriger im Vergleich zu der von mit reinem P(HB-HV)-Extrakt inkubiertem Serum. Darüber hinaus zeigte sie in etwa den gleichen $CH_{50}$-Wert wie Cuprophan.

c) ATP-Freisetzungrstests

In einer weiteren vergleichenden Analyse wurden DLCbeschichtete Stents mit P(HB-HV)-1,0Sh-beschichteten Stents untersucht. Eingesetzt wurde hierzu frisches humanes Plättchenreiches Plasma (PRP). Die Stents wurden mit 450 µl PRP für 5 min. bei 37 °C inkubiert. Die Plättchenaggregation und die Sekretionsfähigkeit wurde durch Einsatz des hoch sensitiven LUMI-AGREGOMETER (CHRONO-LOG Corp., USA) mittels Biolumineszenz gemessen.
Es zeigte sich, dass die Shikonin-beschichteten Stents den funktionellen Status der Plättchen nicht änderten (mit Bezug auf die ATP-Freisetzung), wohingegen die DLC-beschichteten Stents eine ATP-Freisetzung inhibierten.

d) Intramuskulärer Implantations-Test

1. Filme aus lediglich P(HB-HV) und aus P(HB-HV)-Sh wurden gemäß ISO 10993 Teil 6 in die Muskeln von Meerschweinchen implantiert. Die Größe der Filmproben betrug 0,5 x 1 cm mit einer Dicke von 20 bis 30 µm. Für jeden Probentyp wurden zwei Meerschweinchen mit dem Implantat für 7 Tage beobachtet.
Nach 7 Tagen zeigte sich, dass sich um die P(HB-HV)- und P(HB-HV)-Sh-Filme Fibroblastenmonolayers bildeten. Eine Infiltration von Makrophagen und Lymphocyten um diese Implantate herum erwies sich als typische Gewebeantwort nach einer 7-tägigen Implantation. Dennoch konnte gezeigt werden, dass der Grad der Entzündungsreaktion bezüglich des biologisch abbaubaren Polymers mit Shikonin geringer war im Vergleich zu dem reinen P(BB-HV)-Film.
2. Adulte männliche Vistar-Ratten (230 - 250 g) wurden in zwei Gruppen eingeteilt: Den Tieren der Gruppe A (n=6) wurden P(HB-HV)-1,0Sh-beschichtete Stents implantiert, der Gruppe B (n=4) diamantähnlich beschichtete Stents (DLC-Stents) als Kontrolle. Die Stents blieben subkutan über einen Zeitraum von 2 und 6 Wochen in die Rücken der Ratten implantiert, um anschließend subchronische (Kurzzeit-)Effekte zu makroskopisch und mikroskopisch bestimmen (für die einzelnen Zeiträume jeweils n=3 für die Gruppe A und n=2 für die Gruppe B). Betäubt wurden die Tiere mit Natrium-Theopenthal und nach Beendigung der Experimente mit einer Überdosis dieser Substanz eingeschläfert.
Für die histologischen Untersuchungen wurden die Implantate mit umgebendem Gewebe in 12 % Formaldehyd fixiert, in Gelatine eingebettet und mit einer Diamant-Klinge in Schnitte aufgeteilt.
Histologische und makroskopische Untersuchungen zeigten, dass bei beiden Gruppen keine chronisch entzündlichen Fremdkörper-Reaktionen beobachtet werden konnten, jedoch wurden bei den DLC-Stents im umgebenden Gewebe eine beträchtliche Anzahl an Makrophagen sowie einige Kohlenstoff-Partikel nachgewiesen. Die fibröse Kapsel, die sich um die Stents bildete, war bei den P(HB-HV)-1,0Sh-beschichteten Stents bedeutend dünner als bei den DLC-Stents.
Insgesamt war die Stabilität und Biokompatibilität der P(HB-HV)-1,0Sh-beschichteten Stents höher als die der DLC-Stents.
3. In weiteren Versuchen wurden die Stents bei Katzen in die Aorta implantiert. Die Versuchstiere wurden hierzu in zwei Gruppen eingeteilt: Gruppe A (n=2) erhielt P(HB-HV)-1,0Sh-beschichteten Stents, Grupps B (n=2) erhielt DLC-Stents. Adulte weibliche Katzen (2,5 bis 3,5 kg) wurden intramuskulär mit einer 5-prozentigen Ketamin-Lösung (0,65 mg/kg) und einer 0,25-prozentigen Droperidol-Lösung (0,1 mg/kg) betäubt. Die Stents wurden in den Abdomen-Teil der Aorta implantiert. Es wurden keine zusätzlichen Antikoagluantien verabreicht.

18 bis 24 Tage nach der Implantation wurden die Tiere betäubt, die Aorten-Segmente entfernt und in 10 % Formalin fixiert und dehydratisiert. Anschließend wurden die Stentsenthaltenden Segmente in Methylmethacrylat eingebettet, und mittels einer Diamanten-Klinge in Schnitte aufgeteilt.

Für beide Testgruppen konnte keine akute thrombotische Okklusion oder Wucherung des fibrösen Bindegewebes in das Lumen beobachtet werden. Die histologischen Untersuchungen ergaben, dass für die DLC-Stents einige Kohlenstoff-Partikel und eine größere Anzahl an Makrophagen in der die DLC-Stents umgebenden Aortenwand beobachtet wurden. 24 Tage nach der Implantation konnte bei den P(HB-HV)-1,0Sh-beschichteten Stents eine Bildung der Neointima beobachtet werden. Sie war regelmäßiger ausgebildet als bei den DLC-Stents. Anhaltspunkte für eine akute oder eine chronische Entzündung wurden nicht beobachtet.

Zusammenfassung

Durch die Ergebnisse konnte gezeigt werden, dass zum einen die Zusammensetzung aus 25 Gew.-% Polyhydroxyvalerat und 75 Gew.-% Polyhydroxybutyrat sich als optimale Zusammensetzung für die Beschichtung erwies und dass diese Mischung zusammen mit einem bestimmten Shikonin-Gehalt eine hohe Adhäsion an metallischen Oberflächen aufwies. Es konnte auch weiterhin gezeigt werden, dass durch eine wiederholte Expansion des beschichteten Stents die Eigenschaften der Polymerbeschichtung nicht beeinflusst wurden.

Weiterhin konnte ferner gezeigt werden, dass eine Konzentration von Shikonin von bis zu 5 Gew.-% die Adhäsion von menschlichen Blutplättchen an die Polymeroberfläche in vitro praktisch gänzlich inhibierte. Aus diesen Gründen kann Shikonin in geeigneten Konzentrationen verwendet werden, um eine exzessive Zellproliferation zu verhindern.

Eine vergleichende Studie zu den biologischen Eigenschaften eines Films aus reinem P(HB-HV) oder aus P(HB-HV)-Sh in THF in vitro zeigten, dass Shikonin bezüglich der Komplement-Aktivierung und Blutplättchenadhäsion gute kompatible Eigenschaften aufwies.

Durch die Ergebnisse konnte ferner gezeigt werden, dass durch eine Beschichtung mit P(HB-HV)-Sh eine glatte, dünne, fibrozelluläre Schicht um den Stent gebildet wird, was zu einer Reendothelialisierung führt, mit einem ausreichenden Durchmesser für den normalen Blutfluss.

**Patentansprüche**

1. Beschichtungszusammensetzung für eine implantierbare medizinische Vorrichtung, wobei die Beschichtungszusammensetzung zumindest ein Polymer und zumindest einen biologisch aktiven Wirkstoff aufweist, **dadurch gekennzeichnet, dass** der biologisch aktive Wirkstoff Naphthazarin und/oder ein Naphthazarin-Derivat ist.

2. Beschichtungszusammensetzung nach Anspruch 1, **dadurch gekennzeichnet, dass** das Derivat aus der Gruppe enthaltend Shikonin, Alkannin, Arnebin, ausgewählt ist.

3. Beschichtungszusammensetzung nach Anspruch 1 oder 2, **dadurch gekennzeichnet, dass** das Derivat Shikonin ist.

4. Beschichtungszusammensetzung nach einem der Ansprüche 1. bis 3, **dadurch gekennzeichnet, dass** Naphthazarin und/oder Naphthazarin-Derivat in einem Anteil von 0,01 bis 5 Gew.-% enthalten ist.

5. Beschichtungszusammensetzung nach einem der Ansprüche 1 bis 3, **dadurch gekennzeichnet, dass** das Naphthazarin und/oder Naphthazarin-Derivat in einem Anteil enthalten ist, der ausgewählt ist aus der Gruppe umfassend 0,04 Gew.-%, 0,05 Gew.-%, 0,06 Gew.-%, 0,07 Gew.-% , 0,08 Gew.-%, 0,09 Gew.-%, 1 Gew.-%.

6. Beschichtungszusammensetzung nach einem der Ansprüche 1 bis 5, **dadurch gekennzeichnet, dass** das Polymer ausgewählt ist aus der Gruppe: biokompatibles Polymer, resorbierbares Polymer und resorbierbarer Polyester.

7. Beschichtungszusammensetzung nach Anspruch 6, **dadurch gekennzeichnet, dass** der resorbierbare Polyester aus der Gruppe umfassend Polyglykolsäure, Polymilchsäure, Polycaprolakton, Polyhydroxyalkanoate und Copolyester davon, ausgewählt ist.

8. Beschichtungszusammensetzung nach Anspruch 7, **dadurch gekennzeichnet, dass** das Polyhydroxyalkanoat aus der Gruppe umfassend Polyhydroxybutyrat, Polyhydroxyvalerat und Copolyester davon ausgewählt, ist.

9. Beschichtungszusammensetzung nach Anspruch 8, **dadurch gekennzeichnet, dass** der Copolyester ein Polyhydroxybutyrat-Polyhydroxyvalerat-Copolyester ist.

10. Beschichtungszusammensetzung nach Anspruch 9, **dadurch gekennzeichnet, dass** in dem Copolyester Polyhydroxyvalerat in einem Anteil von 20 bis 30 Gew.-%, vorzugsweise von 25 Gew.-%, und Polyhydroxybutyrat in einem Anteil von 70 bis 80 Gew.-%, vorzugsweise von 75 Gew.-%, enthalten ist.

11. Beschichtungszusammensetzung nach einem der Ansprüche 1 bis 10, **dadurch gekennzeichnet, dass** das resorbierbare Polymer und Naphthazarin und/oder Naphthazarin-Derivat in zumindest einem Lösungsmittel gelöst vorliegen.

12. Beschichtungszusammensetzung nach Anspruch 11, **dadurch gekennzeichnet, dass** das Lösungsmittel Dimethylacetamid und/oder Tetrahydrofuran ist.

13. Verfahren zur Beschichtung einer implantierbaren medizinischen Vorrichtung mit den Schritten:

   (a) Aufbringen der Beschichtungszusammensetzung nach einem der Ansprüche 1 bis 12 auf die implantierbare medizinische Vorrichtung,
   (b) Trocknen der implantierbaren medizinischen Vorrichtung.

14. Verfahren zur Beschichtung einer implantierbaren medizinischen Vorrichtung mit den Schritten:

   (a) Aufbringen der Beschichtungszusammensetzung nach einem der Ansprüche 1 bis 12 auf die implantierbare medizinische Vorrichtung,
   (b) Trocknen der implantierbaren medizinischen Vorrichtung und
   (c) Wiederholen der Schritte (a) und (b).

15. Verfahren nach Anspruch 13 oder 14, **dadurch gekennzeichnet, dass** die Beschichtungszusammensetzung aufgesprüht wird.

16. Verfahren nach Anspruch 13 oder 14, **dadurch gekennzeichnet, dass** die Beschichtungszusammensetzung durch Eintauchen der implantierbaren medizinischen Vorrichtung in die Beschichtugszusammens et zung aufgebracht wird.

17. Verfahren zur Beschichtung einer implantierbaren medizinischen Vorrichtung nach einem der Ansprüche 13 bis 16, **dadurch gekennzeichnet, dass** eine Beschichtungszusammensetzung aufgebracht wird, die ein Copolyester aus Polyhydroxybutyrat-Polyhydroxyvalerat in einem Verhältnis von 3:1 und Shikonin in einem Anteil von 0,01 bis 5 Gew.-% aufweist.

18. Verfahren zur Beschichtung einer implantierbaren medizinischen Vorrichtung nach einem der Ansprüche 13 bis 17, **dadurch gekennzeichnet, dass** als implantierbare Vorrichtung ein Stent eingesetzt wird.

19. Verwendung einer Beschichtungszusammensetzung nach einem der Ansprüche 1 bis 12 zur Beschichtung von implantierbaren medizinischen Vorrichtungen.

20. Verwendung von Naphthazarin und/oder eines Naphthazarin-Derivats, insbesondere Shikonin, zur Herstellung einer Beschichtungszusammensetzung für eine implantierbare medizinische Vorrichtung.

21. Verwendung von Naphthazarin und/oder eines Naphthazarin-Derivats, insbesondere Shikonin, für die Beschichtung einer implantierbaren medizinischen Vorrichtung.

22. implantierbare medizinische Vorrichtung, die mit einer Beschichtungszusammensetzung nach einem der Ansprüche 1 bis 12 beschichtet ist.

23. Stent, der mit einer Beschichtungszusammensetzung nach einem der Ansprüche 1 bis 12 beschichtet ist.

**Claims**

1. A coating composition for an implantable medical device, the coating composition comprising at least one polymer and at least one bioactive agent, **characterized in that** the bioactive agent is naphthazarin and/or a naphthazarin derivative.

2. The coating composition as claimed in claim 1, **characterized in that** the derivative is selected from the group comprising shikonin, alkannin, arnebin.

3. The coating composition as claimed in claim 1 or 2, **characterized in that** the derivative is shikonin.

4. The coating composition as claimed in any of claims 1 to 3, **characterized in that** naphthazarin and/or naphthazarin derivatives are present in the content of from 0.01 to 5% by weight.

5. The coating composition as claimed in any of claims 1 to 3, **characterized in that** the naphthazarin and/or naphthazarin derivative is present in a content which is selected from the group comprising 0.04% by weight, 0.05% by weight, 0.06% by weight, 0.07% by weight, 0.08% by weight, 0.09% by weight, 1% by weight.

6. The coating composition as claimed in any of claims 1 to 5, **characterized in that** the polymer is selected from the group: biocompatible polymer, absorbable polymer and absorbable polyester.

7. The coating composition as claimed in claim 6, **characterized in that** the absorbable polyester is selected from the group comprising polyglycolic acid, polylactic acid, polycaprolactone, polyhydroxyalkanoate and copolyesters thereof.

8. The coating composition as claimed in claim 7, **characterized in that** the polyhydroxyalkanoate is selected from the group comprising polyhydroxybutyrate, polyhydroxyvalerate and copolyesters thereof.

9. The coating composition as claimed in claim 8, **characterized in that** the copolyester is a polyhydroxybutyrate-polyhydroxyvalerate copolyester.

10. The coating composition as claimed in claim 9, **characterized in that** in the copolyester polyhydroxyvalerate is present in a content of from 20 to 30% by weight, preferably of 25% by weight, and polyhydroxybutyrate is present in a content of from 70 to 80% by weight, preferably of 75% by weight.

11. The coating composition as claimed in any of claims 1 to 10, **characterized in that** the absorbable polymer and naphthazarin and/or naphthazarin derivative are present dissolved in at least one solvent.

12. The coating composition as claimed in claim 11, **characterized in that** the solvent is dimethylacetamide and/or tetrahydrofuran.

13. A method for coating an implantable medical device comprising the steps:

    (a) applying the coating composition as claimed in any of claims 1 to 12 onto the implantable medical device,
    (b) drying of the implantable medical device.

14. A method for coating an implantable medical device comprising the steps:

    (a) applying the coating composition as claimed in any of claims 1 to 12 onto the implantable medical device,
    (b) drying of the implantable medical device and
    (c) repeating steps (a) and (b).

15. The method as claimed in claim 13 or 14, **characterized in that** the coating composition is sprayed on.

16. The method as claimed in claim 13 or 14, **characterized in that** the coating composition is applied by immersing the implantable medical device into the coating composition.

17. The method for coating an implantable medical device as claimed in any of claims 13 to 16, **characterized in that** a coating composition which includes a copolyester of polyhydroxybutyrate-polyhydroxyvalerate in a ratio of 3:1 and shikonin in a content of from 0.01 to 5% by weight is applied.

18. The method for coating an implantable medical device as claimed in any of claims 13 to 17, **characterized in that** a stent is employed as implantable device.

19. Use of a coating composition as claimed in any of claims 1 to 12 for coating implantable medical devices.

20. Use of naphthazarin and/or of a naphthazarin derivative, in particular shikonin, for producing a coating composition for an implantable medical device.

21. Use of naphthazarin and/or of a naphthazarin derivative, in particular shikonin, for coating an implantable medical device.

22. An implantable medical device coated with a coating composition as claimed in any of claims 1 to 12.

23. A stent coated with a coating composition as claimed in any of claims 1 to 12.

**Revendications**

1. Composition de revêtement pour un dispositif médical implantable, dans laquelle la composition de revêtement présente au moins un polymère et au moins une substance biologiquement active, **caractérisée en ce que** la substance biologiquement active est la naphtazarine et/ou un dérivé de naphtazarine.

2. Composition de revêtement selon la revendication 1, **caractérisée en ce que** le dérivé est choisi dans le groupe constitué de la shikonine, de l'alcannine, de l'arnébine.

3. Composition de revêtement selon la revendication 1 ou 2, **caractérisée en ce que** le dérivé est la shikonine.

4. Composition de revêtement selon l'une des revendications 1 à 3, **caractérisée en ce que** la naphtazarine et/ou le dérivé de naphtazarine est contenu en une proportion de 0,01 à 5 % en poids.

5. Composition de revêtement selon l'une des revendications 1 à 3, **caractérisée en ce que** la naphtazarine et/ou le dérivé de naphtazarine est contenu en une proportion qui est choisie dans le groupe constitué de 0,04 % en poids, 0,05 % en poids, 0,06 % en poids, 0,07 % en poids, 0,08 % en poids, 0,09 % en poids, 1 % en poids.

6. Composition de revêtement selon l'une des revendications 1 à 5, **caractérisée en ce que** le polymère est choisi dans le groupe constitué : les polymères biocompatibles, les polymères résorbables et les polyesters résorbables.

7. Composition de revêtement selon la revendication 6, **caractérisée en ce que** le polyester résorbable est choisi dans le groupe constitué de l'acide polyglycolique, de l'acide polylactique, de la polycaprolactone, des polyhydroxyalcanoates et des copolyesters de ceux-ci.

8. Composition de revêtement selon la revendication 7, **caractérisée en ce que** le polyhydroxyalcanoate est choisi dans le groupe constitué du polyhydroxybutyrate, du polyhydroxyvalérate et des copolyesters de ceux-ci.

9. Composition de revêtement selon la revendication 8, **caractérisée en ce que** le copolyester est un copolyester de polyhydroxybutyrate - polyhydroxyvalérate.

10. Composition de revêtement selon la revendication 9, **caractérisée en ce que** le polyhydroxyvalérate est présent dans le copolyester en une proportion de 20 à 30 % en poids, de préférence de 25 % en poids, et le polyhydroxybutyrate en une proportion de 70 à 80 % en poids, de préférence de 75 % en poids.

11. Composition de revêtement selon l'une des revendications 1 à 10, **caractérisée en ce que** le polymère résorbable et la naphtazarine et/ou le dérivé de naphtazarine sont présents sous forme dissoute dans au moins un solvant.

12. Composition de revêtement selon la revendication 11, **caractérisée en ce que** le solvant est le diméthylacétamide et/ou le tétrahydrofurane.

13. Procédé pour le revêtement d'un dispositif médical implantable comprenant les étapes consistant à :

  (a) déposer la composition de revêtement selon l'une des revendications 1 à 12 sur le dispositif médical implantable,
  (b) sécher le dispositif médical implantable.

14. Procédé pour le revêtement d'un dispositif médical implantable comprenant les étapes consistant à :

  (a) déposer la composition de revêtement selon l'une des revendications 1 à 12 sur le dispositif médical implantable,
  (b) sécher le dispositif médical implantable et
  (c) répéter les étapes (a) et (b).

15. Procédé selon la revendication 13 ou 14, **caractérisé en ce que** la composition de revêtement est pulvérisée.

16. Procédé selon la revendication 13 ou 14, **caractérisé en ce que** la composition de revêtement est déposée par immersion du dispositif médical implantable dans la composition de revêtement.

17. Procédé pour le revêtement d'un dispositif médical implantable selon l'une des revendications 13 à 16, **caractérisé en ce que** l'on dépose une composition de revêtement qui présente un copolyester constitué de polyhydroxybutyrate - polyhydroxyvalérate en un rapport de 3/1 et de la shikonine en une proportion de 0,01 à 5 % en poids.

18. Procédé pour le revêtement d'un dispositif médical implantable selon l'une des revendications 13 à 17, **caractérisé en ce que** l'on met en oeuvre à titre de dispositif implantable une endoprothèse.

19. Utilisation d'une composition de revêtement selon l'une des revendications 1 à 12 pour le revêtement de dispositifs médicaux implantables.

20. Utilisation de la naphtazarine et/ou d'un dérivé de naphtazarine, en particulier de la shikonine, pour préparer une composition de revêtement pour un dispositif médical implantable.

21. Utilisation de la naphtazarine et/ou d'un dérivé de naphtazarine, en particulier de la shikonine, pour le revêtement d'un dispositif médical implantable.

22. Dispositif médical implantable, qui est revêtu d'une composition de revêtement selon l'une des revendications 1 à 12.

23. Endoprothèse qui est revêtue d'une composition de revêtement selon l'une des revendications 1 à 12.

Fig. 1a

Fig. 1b